Europäisches Patentamt

European Patent Office

Office européen des brevets

(19)

(11) Publication number: **0 267 050 B1**

(12) **EUROPEAN PATENT SPECIFICATION**

(45) Date of publication of patent specification: **14.09.94**   (51) Int. Cl.5: **A61K 9/50**, A61K 9/12

(21) Application number: **87309854.5**

(22) Date of filing: **06.11.87**

The file contains technical information submitted after the application was filed and not included in this specification

(54) **Aerosols containg liposomes and method for their preparation.**

(30) Priority: **06.11.86 US 927898**

(43) Date of publication of application:
**11.05.88 Bulletin 88/19**

(45) Publication of the grant of the patent:
**14.09.94 Bulletin 94/37**

(84) Designated Contracting States:
**AT BE CH DE ES FR GB GR IT LI LU NL SE**

(56) References cited:
**EP-A- 0 158 441        EP-A- 0 172 007**
**EP-A- 0 190 926        WO-A-86/04233**
**WO-A-86/06959        WO-A-87/01586**
**GB-A- 2 136 762        US-A- 3 594 476**

**CHEMICAL ABSTRACTS, vol. 99, no. 22, November 1983, Columbus, OH (US); L.B. MARKS et al., p. 343, no. 181351c&NUM;**

**JOURNAL OF INFECTIOUS DISEASES, vol. 158, 1988; p. 443-448&NUM;**

**REVIEWS IN PERINATAL MEDICINE, vol. 4, 1981; p. 337-379&NUM;**

(73) Proprietor: **RESEARCH DEVELOPMENT FOUNDATION**
**402 North Division Street**
**Carson City Nevada 89703 (US)**

(72) Inventor: **Knight, Jack Vernon**
**11735 Green Bay Drive**
**Houston, TX 77024 (US)**
Inventor: **Gilbert, Brian E.**
**5171 Jason**
**Houston, TX 77096 (US)**
Inventor: **Wilson, Samuel Z.**
**4326 Briarbend**
**Houston, TX 77035 (US)**
Inventor: **Six, Howard R.**
**5138 Karenbeth**
**Houston, TX 77084 (US)**
Inventor: **Wyde, Philip R.**
**11002 Braewick**
**Houston, TX 77096 (US)**

(74) Representative: **Wilkinson, Stephen John et al**
**Stevens, Hewlett & Perkins**
**1 St. Augustine's Place**
**Bristol BS1 4UD (GB)**

EP 0 267 050 B1

**Description**

Field of the Invention

The field of the invention is small particle aerosol liposomes and liposome-drug combinations advantageous for medical use.

Background of the Invention

Small particle aerosol is defined as a colloid system in which the continuous phase is a gas, and the majority of particles are less than 5 $\mu$m in diameter with an aerodynamic mass median diameter ranging from 1 to 3 $\mu$m. The advantage of such a discretely sized population of particles is that, because of their small size and low settling velocities, they will penetrate when inhaled into the lower respiratory tract in substantial percentages. For example, 1.5 $\mu$m particles will deposit 46 percent of the total inhaled in the lung and another 36 percent in nose and upper air passages. Such uniform deposition will permit treatment of lesions at any level of the respiratory tract (Gilbert, B. E., Wilson, S. Z., and knight, V., 1986, Ribavirin Aerosol Treatment of Influenza Virus Infections. In: Options for the Control of Influenza. UCLA Symposium on Molecular and Cellular Biology. Alan R. Liss, Inc., New York, New York, p. 343.)

Small particle aerosol treatment delivers a high dose of drug to the epithelium of the respiratory tract in amounts largely unachievable by other routes of administration (Knight, V. 1973, Airborne Transmission and Pulmonary Deposition of Respiratory Viruses. In: Viral and Mycoplasmal Infections of the Respiratory Tract. V. Knight, ed. Lea and Febiger, Philadelphia, Pennsylvania, p. 1). There is a subsequent steady rate of absorption of drug into the systemic circulation.

Dried phospholipids placed into an aqueous environment will spontaneously associate into multilamellar structures that function as permeability barriers. These lipid vesicles, termed liposomes, are composed of aqueous compartments separated from each other and the external medium by a series of closed concentric lipid bilayers. The composition of the aqueous compartments is the same as the medium in which the liposomes were formed; this makes it possible to entrap a wide variety of materials within the lipid bilayers. Entrapped markers can be released by a variety of lytic agents in a manner analogous to natural membranes. Since liposomes may be prepared from substances found in normal cell membranes, they are perceived as nontoxic to mammalian host; and recent studies in humans and laboratory animals have supported this concept.

The ability to encapsulate water soluble compounds in liposomes led to speculation that they might be useful clinically as carriers of drugs. This expectation has not been fully realized for wager soluble drugs. However, recent studies with water insoluble anticancer and antimicrobial compounds have suggested that liposomes may be ideally suited for delivery of this type of drug. The amounts of drug associated with liposomes are high, and release does not occur until the membranes are destroyed either by mechanical means or by biodegradation, thus allowing a more controlled release of the drug over time. Moreover, in laboratory animals the use of liposomes actually reduced toxic effects observed with the drug alone.

Liposome-drug compounds are heterogeneous in size ranging from less than 1 $\mu$m up to 10 $\mu$m in diameter and have been given in relatively large oral or intravenous doses.

Description of the Prior Art

Applicants are unaware of any prior art disclosing, suggesting, or teaching small particle aerosol liposomes and liposome-drug combinations for medical use or that the heterogeneous size of small liposomes and liposome-drug combinations can be reduced to a more homogeneous population of small liposome particles (<1 $\mu$m in diameter). The size of the aerosol particle is controlled by the operating characteristics of the aerosol generator or nebulizer without any loss in effectiveness thereof. One to several liposomes or liposome-drug particles (<1 $\mu$m in diameter) may be contained in a single aerosol droplet (1-3 $\mu$m, aerodynamic mass median diameter) depending on the concentration of liposome material in the preparation that is to be nebulized.

Liposomes are effective carriers for the introduction of various agents into cells in in vitro experiments. A large number of scientific papers have been published describing liposomes as carriers for both water-soluble and lipid-soluble agents. Several hundred different substances have been entrapped in liposomes. For a further description of liposomes, their preparation and application, reference is made to American Laboratory, pages 125-135, October, 1985.

The following articles and patents are representative of the prior art:

AMA Drug Evaluation, 5th Edition, pp. 1162-1166

Annals NYAS, "Liposomes in Therapeutic and Preventive Medicine: The Development of the Drug-Carrier Concept", G. Gregoriadis, 1978, 308:343-65

Clinical Science and Mol. Med., "Tissue and Hepatic Subcellular Distribution of Liposomes Containing Bleomycin after Intravenous Administration to Patients with Neoplasms", A. W. Segal, G. Gregoriadis, J. P. Lavender, D. Tarin, T. J. Peters, 1976, 51:421-25

J. Infect. Dis., "Liposomal Amphotericin B for the Treatment of Systemic Fungal Infections in Patients with Cancer: A Preliminary Study", G. Berenstein-Lopez, V. Fainstein, R. Hopfer, K. Mehta, M. Sullivan, M. Keating, M. Rosenblum, R. Mehta, M. Luna, E. Hersh, J. Reuben, R. Juliano, G. Bodey, 1985, 151:704-10

Infect. Immun., "Effect of Liposomal Amphotericin B on Murine Macrophages and Lymphocytes", R. Mehta, K. Mehta, G. Lopez-Berenstein, R. Juliano, 1985, 47:429-33

The Lancet, "Artificial Surfactant Therapy in Hyaline-Membrane Disease", T. Fujiawara, H. Maeta, S. Chida, T. Morita, Y. Watabe, T. Abe, 1980, 1:55-59

Pediatrics, "Hyaline Membrane Disease Treated with Bovine Surfactant", J. A. Smyth, I. L. Metcalfe, P. Duffty, F. Possmayer, M. H. Bryan, G. Enhorning, 1983, 71:913-17

Pediatrics, "Isolation of Human Surfactant from Amniotic Fluid and a Pilot Study of its Efficacy in Respiratory Distress Syndrome", M. Hallman, T. A. Merritt, H. Schneider, B. Epstein, F. Mannino, D. Edwards, L. Gluck, 1983, 71:473-82

Pediatric Res., "Nebulization of Sonicated Phospholipids for Treatment of Respiratory Distress Syndrome of Infancy", H. H. Ivey, J. Kattinwinkel, S. Roth, 1977, 11:301-14

The Lancet, "Dry Artificial Lung Surfactant and its Effect on Very Premature Babies", C.J. Morley, A.D. Banham, N. Miller, J.A. Davis, 1981, 1:64-68

The Lancet, "Controlled Trial of Artificial Surfactant to Prevent Respiratory Distress Syndrome", H.L. Halliday, G. McClure, M. Reid, T.R.J. Lappin, C. Meban, P.S Thomas, 1984, 1:476-78

U.S. Patent No. 4,370,349 disclosing a process for preparing a freeze-dried, potential liposome mixture

U.S. Patent No. 3,873,720 disclosing an aqueous mixture of fat, carbohydrate and amino acids emulsified with the aid of long chain fatty acid or its basic amino acid salts and egg-yolk phospholipids.

U.S. Patent No. 4,073,943 disclosing parental administration of water-insoluble pharmacologically active agents in lipoyd phase.

U.S. Patent No. 4,168,308 disclosing parental administration of water-insoluble pharmacologically active agents in lipoyd phase.

U.S. Patent No. 4,536,519 directed to an emulsifying agent and emulsified cosmetics.

U.S. Patent No. 4,563,354 disclosing parental administration of oil and water emulsions

US-A-3,594,476 describes the preparation of aqueous aerosols, containing lecithin, of sufficiently small particle size (submicron diameter) to be suitable for transmission, by inhalation, to the alveoli of the lung.

EP-A-0158441 discloses pro-liposome compositions which, on addition to excess water, spontaneously form dispersions of liposomes.

EP-A-0172007 discloses a method for preparing stable liposomes with aqueous soluble medicaments by lyophilization of lipid prior to hydration with a medicament-containing solution.

EP-A-0190926 discloses the preparation of liposomes by preparing a solution of a lipid component in an organic solvent, combining this with water and passing the resulting mixture under pressure through an orifice to produce an aerosol spray containing liposomes.

WO 86/04233 discloses an aerosol formulation containing a chlorofluorocarbon propellant, glycerol phosphatides and a drug for topical application or for inhalation.

WO 86/06959, which is a prior art document according to Art.54(3)EPC, discloses a liposome-based drug delivery system for inhalation wherein the phospholipid composition of the liposomes is formulated to produce a selected rate of drug release from the liposomes when administered to the respiratory tract.

WO 87/01586, which is a prior art document according to Art.54(3)EPC, discloses an aerosol comprising liposomes suspended in a carrier fluid, the liposomes having entrapped therein a therapeutic or diagnostic agent.

Pediatric Research, Vol. 17, No. 9, (1983), pages 742 to 749 discloses the production of surface active aerosols from aqueous dispersions of mixed lipids using ultrasonic and jet nebulizers.

## Summary of the Invention

The present invention is directed to small particle aerosol droplets containing one or more liposome particles and liposome-drug combinations having advantageous properties for medical use. Small particle aerosol, as used herein, is a colloid system in which the continuous phase is a gas, and the majority of particles (i.e., more than 50% of the total number of particles) are less than 5 $\mu$m in diameter with an aerodynamic mass median diameter ranging from 1 to 3 $\mu$m. The particle size of the liposomes and the liposome-drug compositions are processed by the aerosol nebulizer to substantially homogeneous sizes of less than one micron diameter; and these small liposome and liposome-drug particles retain their pharmacological activity.

Small particle aerosol treatment by liposomes alone is advantageous since liposomes can closely mimic pulmonary surfactant and may repair defects in this system that have developed for a variety of reasons.

The drugs to be given by liposome-drug combinations range widely as does the dosage. In general, the drugs in recommended dosages for non-aerosol liposome-drug combinations of the prior art can be used for the small particle aerosol liposome drug-combinations without the disadvantages of the prior art liposome-drug combinations. The amount of the drug in the liposome-drug combination aerosol is controlled by the concentration of drug in the reservoir of the aerosol generator. Also, the amount of drug employed depends on the duration of treatment, drug used and the like. For example, dosage for 24 hours can range from less than a nanogram to a few grams depending on need, toxicity, biological and chemical properties of the drug, and other factors. Liposome-drug combinations can include both aqueous and lipid soluble drugs.

## Description of Presently Preferred Embodiments

As previously set forth, the present invention is directed to small particle aerosol liposomes and liposome-drug combinations, to methods of generating aerosols of them, and to methods of treating patients with them. As the term "small particle aerosol" is used herein, it is defined as a colloid system in which the continuous phase is a gas, and the majority of particles are less than 5 $\mu$m in diameter with an aerodynamic mass median diameter ranging from 1 to 3 $\mu$m. Liposome-drug combinations of the prior art are heterogeneous in size ranging from less than 1 $\mu$m up to 10 $\mu$m in diameter and, have been given to patients in relatively large oral or intravenous doses. Such dosage results in high and potentially toxic plasma concentrations of drugs, but low concentrations on the respiratory epithelium. Unexpectedly, the heterogeneous lipsomes and the liposome-drug combinations can readily be converted to a more homogeneous small size by an aerosol nebulizer without any loss in effectiveness of the liposomes and liposome-drug combinations. Any type of aerosol nebulizer can be used which so reduces the size of the liposome. Examples of nebulizers that can be used include Puritan Bennett Models Nos. 1920 and 1917 which are both commercially available. Advantageously, the small particle aerosol liposomes and liposome-drug combinations, when inhaled, provide a high concentration on the respiratory epithelium and a steady rate of absorption into the circulation without the hazard of peak levels that may be associated with large oral or intravenous doses of drug, and deliver a high dose of drug to the epithelium of the respiratory tract in amounts largely unachievable by other routes of administration. As previously mentioned, the advantage of such a discretely sized population of particles is that, because of their small size and low settling velocities, they will penetrate when inhaled into the lower respiratory tract in substantial percentages. For example, 1.5 $\mu$m particles will deposit 46% of the total inhaled in the lung and another 36% in the nose and upper air passages. Such uniform deposition permits treatment of lesions at any level of the respiratory tract and also provides an interface into the cell without the problems and disadvantages associated with oral and intravenous injections.

The following Table 1 sets forth representative examples of water and lipid soluble drugs that may be administered in liposome small particle aerosols.

## Table 1

| Antiasthma | Antiarrhythimic | Tranquilizers |
|---|---|---|
| metaproterenol | propanolol | chlorpromazine |
| aminophylline | atenolol | benzodiazepine |
| theophylline | verapamil | butyrophenones |
| terbutaline | captopril | hydroxyzines |
| Tegretol | isosorbide | meprobamate |
| ephedrine | | phenothiazines |
| isoproterenol | | reserpine |
| adrenalin | | thioxanthines |
| norepinephrine | | |

| Cardiac glycosides | Hormones | Steroids |
|---|---|---|
| digitalis | antidiuretic | prednisone |
| digitoxin | corticosteroids | triamcinolone |
| lanatoside C | testosterone | hydrocortisone |
| digoxin | estrogen | dexamethasone |
| | thyroid | betamethosone |
| | growth | prednisolone |
| | ACTH | |
| | progesterone | |
| | gonadotropin | |
| | mineralocorticoid | |

| Antihypertensives | Antidiabetic | Antihistamines |
|---|---|---|
| apresoline | Diabenese | pyribenzamine |
| atenolol | insulin | chlorpheniramine |
| | | diphenhydramine |

| Antiparasitic | Anticancer | Sedatives & Analgesic |
|---|---|---|
| praziquantel | azathioprine | morphine |
| metronidazole | bleomycin | dilaudid |
| pentamidine | byclophosphamide | codeine |
| | adriamycin | codeine-like synthetics |
| | daunorubicin | demerol |
| | vincristine | oxymorphone |
| | | phenobarbital |
| | | barbiturates |

| Antibiotic | Immunotherapies | Vaccines |
|---|---|---|
| penicillin | interferon | influenza |
| tetracycline | interleukin-2 | respiratory syncytial |
| erythromycin | monoclonal antibodies | virus |
| cephalothin | gammaglobulin | Hemophilus influenza |
| imipenem | | vaccine |
| cefofaxime | **Antifungal** | |
| carbenicillin | | **Antiviral** |
| vancomycin | amphotericin B | |
| gentamycin | myconazole | acyclovir and deriva- |
| tobramycin | muramyl dipeptide | tives |
| piperacillin | clotrimazole | Winthrop-51711 |
| moxalactam | | ribavirin |
| amoxicillin | **Antihypotension** | rimantadine/amantadine |
| ampicillin | | azidothymidine & deriva- |
| cefazolin | dopamine | tives |
| cefadroxil | dextroamphetamine | adenine arabinoside |
| cefoxitin | | amidine-type protease |
| other aminoglycosides | | inhibitors |
| | | **Other** |
| | | cell surface receptor |
| | | blockers |

Liposome Preparation

Liposomes and liposome-drug combinations may be prepared in any suitable manner, for example, as described in American Laboratory, pp. 125-135, October, 1985, and U.S. Patent No. 4,370,349, Evans, et.

al., January 25, 1983. These publications amply document that a variety of amphipathic lipids are suitable for preparing of liposomes for use in this invention.

Suitable lipids include the phospholipids, for example the natural lecithins derived from egg-yolk or soya-bean, sphingomyelin derived from beef brain or synthetic lecithins, for example dimyristoyl-phosphatidylcholine, dipalmitoyl-phosphatidylcholine or distearoyl-phosphatidylcholine, or unsaturated synthetic lecithins, for example, dioleyl-phosphatidylcholine or dilinoleyl-phosphatidylcholine. Either a single phospholipid or a mixture of phospholipids may be used. Sterols, for example, cholesterol or ergosterol, may be added to increase stability of the liposomal bilayers and lipids possessing a positive or negative change, for example, phosphatidylethanolamine, beef brain ganglioside or phosphatidic acid, may be used to render the appropriate charge to the liposome and to increase the size of the aqueous compartments. Mixtures of lipids may be used to render the liposomes more fluid or more rigid and to increase or decrease permeability characteristics.

Liposomes can be prepared by a variety of methods. These procedures have in common the dispersal of a phospholipid or mixture of lipids into a suitable container and the removal of an organic solvent, for example, ether, chloroform, or T-butanol, by methods such as evaporation, rotary evaportion under vacuum or lyophilization with commercially available freeze-drying equipment. Dispersing the resulting lipid film or dry lipid powder in an aqueous medium, for example, distilled water, isotonic saline or buffered solutions will result in the formation of liposomes. For example, phosphatidylcholine is dissolved in re-distilled T-butanol and transferred to a bottle. The solution is frozen and the solvent is removed under vacuum using a commercial freeze-dryer. Sterile pyrogen-free distilled water is added to the freeze dried powder and the bottle shaken to disperse the powder. The resulting milky suspension can be examined microscopically and the suspension shown to contain liposomes that are heterogenous in size ranging from less than 1 $\mu$m up to 10 $\mu$m.

Any biologically active compound may be associated with liposomes. Whether the compound is associated with the lipid portion of the liposomes or resides in the aqueous compartments is dependent upon the physical and chemical properties of the compound of biological interest. It is understood that the procedures used for preparing liposome-drug combinations are not restricted under this invention, any procedure that results in liposomes would be applicable. For purposes of disclosure, three general methods of producing liposomes ore described below. They illustrate that regardless of chemical and physical properties a wide array of biologically active compounds can be associated with liposomes and that such liposomes are applicable to delivery by small particle aerosol.

Method I may be used to incorporate lipid soluble or lipid-bound biologically active compounds. For example, egg lecithin (phosphatidylcholine) or similar phospholipids dissolved in an organic solvent Is transferred to a suitable flask or bottle. The desired lipid soluble compound is added, the solution is frozen and the solvent is removed using a commercial freeze-dryer. Liposomes are formed by the addition of a suitable aqueous medium, for example, sterile distilled water, isotonic saline or a buffered solution followed by vigorous shaking of the container. It Is recognized that the phospholipid or mixture of phospholipid used to prepare the liposomes can be altered to increase or decrease the lipid solubility of the active compound as desired and that solvents such as chloroform, n-butanol, t-butanol, or pyridine may be used to promote interaction of the compound and phospholipid. The specific procedure can be tailored to accommodate the individual properties of specific compounds.

Method II may be used to incorporate water soluble biologically active compounds. For example, egg lecithin dissolved in redistilled T-butanol is transferred to a suitable flask or bottle. The solution is frozen and the solvent is removed using a commercial freeze-dryer. Compounds to be entrapped in the liposomes are then added to the dry powder in aqueous medium and the liposomes are formed by vigorous shaking of the vessel to disperse the dry powder. Under these conditions, entrapment of 4-5% of the water soluble compound is expected but addition of a sterol and/or a charged amphiphile in the lipid mixture can increase the entrapment efficiency up to approximately 30%. A dry compound that remains "free" in the external medium can be separated by a variety of procedures, for example centrifugation, molecular sieve chromatography or dialysis. The desirability of removing the free compound or not is dependent upon the intended medical use, the biologic activity of the compound, the desired dose and the quantities incorporated into the liposomes.

Method III may be used to incorporate biologically active compounds without regard to their solubility characteristics. In this procedure the compound is covalently attached to a lipid with the result that the lipid moiety will associate with the liposome and anchor the compound to the liposomal bilayers. Phosphatidylethanolamine and palmitic acid have been utilized for this purpose but a variety of lipids may be applicable to this method. In this procedure the compound and a lipid derivative capable of derivatizing the compound, for example, the N-hydroxy-succinimide ester of palmitic acid, N-succinyl-phosphatidylethanolamine, or alternatively phosphatidylethanolamine in the presence of a dehydrating agent such as N- N'-dicyclohexylcarbodiimide, are mixed in a suitable solvent and allowed to react. The lipid derivative of the compound is then purified and incorporated into liposomes. For example, egg lecithin or similar phospholipid and appropriate quantities of lipid derivatized compound are dissolved in an organic solvent and added to a suitable flask or bottle. The solution is frozen and the solvent removed in a freeze-dry apparatus. Liposomes then formed by addition of suitable aqueous medium to the dry powder, followed by vigorous shaking of the container. It is recognized that wide variety of chemical reactions can be utilized to prepare lipid derivatives of biologically active compounds and that alternative procedures will be suitable, if the resulting derivative can be associated with liposomes and if the biological activity of the compound has not been irreversibly destroyed by the process. It is also recognized that lipid derivatives of some compounds, for example peptides, proteins or hormones may be efficiently incorporated when added in the aqueous medium rather than to the organic solvent.

Liposome-Drug Combination

Example 1

One aspect of the invention, and the present example are directed to liposome-drug combinations where the drug is enviroxime and made according to Method I.

For enviroxime containing liposomes, phosphatidylcholine (450 mg) was added to a 500 mL flask in chloroform (30 mL) and 120 mg of enviroxime was added to the solution. The solvent was removed under vacuum and the lipid-drug mixture dissolved in 60 mL of T-butanol. The solution was frozen and the solvent removed in a commercial freeze-dry apparatus. Liposomes were prepared by mechanically shaking the dried residue in 30 mL of sterile water. Liposomes prepared by this procedure were examined microscopically and they were found to be heterogeneous in size ranging from less than 1 $\mu$m up to 10 $\mu$m in diameter. The preparation was then placed in a small particle aerosol generator that used a Collison nebulizer for formation of the small particles. During passage through the nebulizer, the liposomes were reduced in size so that most liposomes were less than 1 $\mu$m in diameter and many less than 0.1 $\mu$m in diameter. The size of the particles containing enviroxime-liposomes delivered to the patient is controlled by the operational characteristics of the aerosol generator. The majority of these particles were less than 5 $\mu$m in diameter with an aerodynamic mass median diameter ranging from about 1 to 3 $\mu$m. Any type of aerosol nebulizer can be used which so reduces the size of the liposome, a number of which are available on the market. For example, Puritan Bennett nebulizer model No. 1920 or model No. 1917 can be used by placing the liposomes or liposome-drug combinations in the reservoirs of these nebulizers. Accordingly, no further description of the nebulizers is deemed necessary or given.

We have found that the enviroxime-containing liposomes as initially made (as present in the aerosol reservoir at the start of operation of the aerosol generator) are very heterogeneous in size, ranging from 0.7 $\mu$m down to less than 0.03 $\mu$m in diameter. Following processing by the small particle aerosol generator (SPAG), observations of the liposomes in the aerosol reservoir after 60 minutes of operation and also of liposomes in an aerosol sample collected in an All Glass Impinger (AGI) after 15 minutes of operation show that the liposomes become more homogeneous in size, with the larger ones being less than about 0.35 $\mu$m in diameter. The generation of small particle size does not change the structure of the liposomes and the liposomes were found to have retained their liposome characteristics and to be multi-lamellar liposomes of "classical" structure.

Enviroxime is a substituted imidazole with exceptional potency against all rhinoviruses tested: inhibitory concentrations generally range from 0.3 to 0.9 $\mu$g/mL. Maximum tolerated concentrations for cells in culture range from 4 to 100 $\mu$g/mL, resulting in therapeutic ratios ranging from 50 to 100. Enviroxime is active also against polioviruses, echoviruses and coxsackieviruses. The drug was discovered by Eli Lilly and Company.

Enviroxime is only slightly soluble in water (1-2 $\mu$g/mL) and this has posed a problem in medical use of the drug. This difficulty is overcome by preparing liposomes of enviroxime (1-8 mg/mL) and phosphatidylcholine (15 mg/mL) in particles small enought to be administered as a small particle aerosol. By this methodology, doses of 6 to 12 mg/hr can be given to the respiratory tract.

Treatment periods of one hour to four hours per day are satisfactory. Daily dosage is set forth in Table 2 for an average-sized adult (70 kg):

Table 2

| Duration | Mg/Hr | Total Dose (mg) | Mg/Kg/Day |
|---|---|---|---|
| 1 hr | 6 | 6 | 0.08 |
| 1 hr | 12 | 12 | 0.16 |
| 2 hr | 6 | 12 | 0.16 |
| 2 hr | 12 | 24 | 0.32 |
| 4 hr | 6 | 24 | 0.32 |
| 4 hr | 12 | 48 | 0.64 |

Table 2 reveals that all proposed doses are substantially less than 1 mg/kg/day. Tolerance studies in animals summarized below show lack of untoward effects with doses many-fold larger than those proposed above.

Animal Tolerance Studies: Pharmacokinetics

Effects in vitro on muscle. Muscle from many organs of rats was tested with $10^{-5}$ to $10^{-8}$ M enviroxime. It did not activate adrenergic, histamine, prostaglandin E2 and a number of other biologic receptors. There was some non-competitive antagonism of dose response curves similar to that of potassium chloride.

Effects on electrolytes. At dose of 25 mg/kg and above there was significant oliguria in rats. The oliguria was associated with increases in potassium in serum but not sodium.

Effects in mice. Doses of 50 mg/kg orally produced no detectable effects. At higher doses, 100-400 mg/kg, there was increasing leg weakness, decreased motor activity and gait disturbances. The effect of high doses was rapid, occurring within a few minutes. A number of other tests were performed with oral doses and in general doses up to 100 mg/kg were well tolerated. Of particular interest was the finding that there was no immune suppressive activity of enviroxime on primary antibody response in mice.

Effects on cats and dogs. There was no significant cardiovascular effects on cats. There was some stepwise depression of diastolic blood pressure in dogs following 1, 3, and 10 mg/kg I.V. doses. Plasma concentrations of enviroxime were 3.0, 8.9 and 19 $\mu$g/mL following these I.V. doses. Bioassay (antiviral) and biochemical assay of blood yielded similar results.

The above studies, and others, are acceptable as evidence of adequate safety for human use and human studies.

Example 2

In this aspect of the invention and example ribavirin is the drug in the liposome-drug combination which is made by Method II.

For ribavirin containing liposomes, 450 mg of egg phosphatidylcholine was added to a 500 mL round bottom flask and the organic solvent removed under vacuum. Ribavirin (600 mg) in 30 mL of aqueous medium (sterile phosphate buffered saline) was added and the liposome prepared by mechanical shaking. Again, the ribavirin-liposomes were heterogeneous in size but passage through a Collison nebulizer reduced the ribavirin-liposomes to sizes as set forth in Example 1.

Example 3

In this aspect of the invention and example, Method III, methotrexate is the drug in the liposome-drug combination. (Hashimoto, K., Loader, J. E., and Kinsky, S. C., 1985, Synthesis and Characterization of Methotrexate-dimyristoylphosphatidylethanolamine Derivatives and the Glycerophosphorylethanolamine Analogs. Biochim. Biophys. Acta 816:163-168; Hashimoto, K., Loader, J. E., Knight, M. S., and Kinsky, S. C., 1985, Inhibition of Cell Proliferation and Dihydrofolate Reductase by Liposomes Containing Methotrexate-dimyristoylphosphatidylethanolamine Derivates and by the Glycerophosphorylethanolamine Analogs. Biochim. Biophys. Acta 816:169-178).

Methotrexate (40 $\mu$mol) is dissolved in 0.8 mL of a 1:1 volume mixture of chloroform and methanol (hereafter abbreviated C/M) containing triethylamine (240 $\mu$mol). The following were then added sequentially to this solution while stirring; dimyristoyl-phosphatidylethanolamine (120 $\mu$mol) dissolved in 5.6 mL, of C/M; N-hydroxysuccinimide (200 $\mu$mol) dissolved in 0.8 mL of C/M; N,N'-dicyclohexylcarbodiimide (200 $\mu$mol) dissolved in 0.8 mL of C/M. After incubation for 3 hours at room temperature, the reaction mixture was taken to dryness by rotary evaportion under reduced pressure at 40°C, and the residue was redissolved in 2 mL of C/M.

Chromatographic separation of the methotrexate pnosphatidylethanolamine derivatives was accomplished by streaking 250 µL of this fraction on each of 8 analytical thin-layer plates (Silica gel 60 F-254, 0.25 mm, Brinkmann Instruments, Inc., Westbury, New York). The plates were developed in a solvent system of chloroform/methanol/water (65:30:5 by vol.). After development, four yellow bands (I-IV) were visible that also gave a positive test for phosphate when sprayed with an acid molybdate reagent (Applied Science, Deerfield, Illinois). These bands had approximate $R_F$ values of 0.18 (I), 0.28 (II), 0.39 (III), and 0.49 (IV), whereas the $R_F$ for the unreacted methotrexate band, which did not stain for phosphate was 0.06. Band I which was shown to possess full biologic activity was scraped from the plates and suspended in 5 mL of methanol. After centrifugation (750 x g for 10 min. at 4°C), the yellow supernatant was recovered, and the pelleted silica gel particles were reextracted with another 5 mL of methanol. Ten mL of chloroform was added to the combined supernatants, and this solution was layered over a 50 mm high bed of Unisil (Clarkson Chemical Co., Williamsport, Pennsylvania) at the bottom of a 1 x 20 cm column. The Unisil previously had been washed extensively with chloroform, followed by C/M. The yellow compound (designated MTX-CMPF) was subsequently eluted by passage of 20 mL of C/M. The eluate was taken to dryness, and the residue was redissolved in 5 mL of C/M and stored at -20°C.

Liposomes were generated from dried lipid films containing dioleoyl-phosphatidylcholine (DOPC), cholesterol, and dicetylphosphate in a molar ratio of 2:1.5:0.2, respectively. The film was also supplemented with 2.5 mol% of MTX-DMPE I on the basis of phosphate content. The lipid films were dispersed by vortexing in sufficient balanced salt solution to give a 10 mM liposomal (DOPC) suspension. MTX-DMPE I had a phosphate methotrexate ratio of 1. Accordingly, the final methotrexate density in liposomes prepared with the derivative was 2.5 mol% methotrexate.

Further examples, numbered 4 to 23 are shown in Table 3 in which the preferred methodology for preparation in liposomes, concentration of drug in the aerosol reservoir and the amount of drug delivered in aerosol in a specified period of time are shown. The delivered doses approximate single doses of drug which might be given by oral or parenteral routes of administration.

Table 3

| Suggested Delivered Dose of Representative Liposome-Containing Compounds as Delivered by Small Particle Aerosol | | | | | |
|---|---|---|---|---|---|
| Example No. | Compound | Method[1] | Concentration in Reservoir (mg/mL) | Duration of Treatment | Estimated Delivered Dose[2] (mg) |
| 4 | Amantadine | 1 | 4 | 12 hrs | 172 |
| 5 | Digtoxin | 1 | 1 | 20 min | 1 |
| 6 | Isosorbide | 1 | 0.5 | 3 hrs | 5 |
| 7 | Estrogens | 1 | 0.8 | 8 hrs | 23 |
| 8 | Diabinese | 1 | 6 | 10 hrs | 216 |
| 9 | Amphotericin B | 1 | 20 | 30 min | 36 |
| 10 | Prednisone | 1 | 8 | 60 min | 29 |
| 11 | Interferon | 1 | $5 \times 10^6$ units/mL | 20 min | $6 \times 10^6$ units |
| 12 | Isoproterenol | 2 | 2 | 20 min | 2 |
| 13 | Apresoline | 2 | 15 | 20 min | 18 |
| 14 | Gentamycin | 2 | 100 | 30 min | 180 |
| 15 | Propanolol | 2 | 3 | 20 min | 4 |
| 16 | Dopamine | 2 | 150 | 20 min | 180 |
| 17 | Chlorpromazine | 2 | 60 | 20 min | 72 |
| 18 | Diphenhydramine | 2 | 15 | 20 min | 18 |
| 19 | Morphine sulfate | 2 | 10 | 20 min | 12 |
| 20 | Demerol | 2 | 75 | 20 min | 90 |
| 21 | Acyclovir | 2 | 20 | 12 hrs | 864 |
| 22 | Amantadine | 2 | 20 | 12 hrs | 864 |
| 23 | Influenza vaccine | 3 | 40 ug HA/mL | 20 min | 48 ug HA |

1. Methods:
1) As per Method I for water insoluble compounds;
2) As per Method II for water soluble compounds;
3) As per Method III for covalent attachment of compounds to the surface of the lipid bilayer.
2. Estimated dose based on a 60% of maximum efficiency of aerosol generation and a 10 L minute minute volume for a 70 kg adult, and on currently given dosages.

Also, combinations of more than one drug can be combined with small particle liposome aerosols.

Advantageously, small particle aerosol treatment with liposome-drug combinations leads to deposition of drug and liposomes throughout the respiratory tract in substantial concentrations that can treat infections that are localized to the respiratory tract. In the case of viruses, the infection is localized to respiratory epithelial cells. In the case of bacteria or fungi, the diseases will be contained in inflammatory exudates and alveoli and in other anatomical spaces in the lung and within tissues of the lung at various locations. Aerosolized liposome-drug will be deposited on these sites.

In the case of lung tumors of primary or secondary origin, the tumor masses would be the site of deposition of aerosol liposome-anti cancer drugs.

In the case of asthma, aerosolized liposome bronchodilator agents would be deposited throughout the bronchial tree at sustained levels for extended periods of time to provide optimum therapeutic effect.

In the case of psychiatrically useful drugs, hormones, or cardioactive agents, systemic absorption following aerosol liposome-drug administration would occur at an even rate without high peaks in plasma concentration, thus avoiding potential toxicity and prolonging therapeutic effect.

Aerosol liposomes alone may replace natural surfactants in the lung of victims of drowning, chemical inhalational poisoning, and in premature infants deficient in surfactant.

Influenza or other vaccines can be given conveniently in small particle aerosol liposomes deposited directly on immunoreactive cells in the lung to elicit locally protecting immune responses. Humoral antibody may also be so stimulated.

Incorporation of some toxic agents such as amphotericin B into liposomes in aerosolized form retards their absorption into cells of the respiratory tract and reduces the toxic effect of the toxic agents without reducing their therapeutic effect.

10

Also, incorporation of polypeptides, oligonucleotides, enzymes, or other compounds which might be destroyed or inactivated by localized enzymes may be protected from this effect when incorporated in small particle aerosolized liposomes thus increasing their therapeutic effect.

Thus, while specific examples of a variety of small particle liposomes and liposome-drug combinations have been given for purposes of disclosure, the present invention is applicable to all drugs or combinations of drugs which can be incorporated in small particle liposome aerosols for a wide variety of disease. Also, as previously mentioned, the dosage of the small particle liposome-drug combinations vary widely depending on the drug, duration of treatment and the like.

Accordingly, the present invention is well suited and adapted to attain the ends and carry out the objects and has the advantages and features set forth as well as others inherent therein. While presently preferred embodiments, uses, and treatments of various disease have been given for the purpose of disclosure, changes therein, modifications thereto, and other uses and treatments of disease can be made which are within the scope of the invention as defined by the following claims.

**Claims**

1.  Aqueous aerosol droplets containing one or more liposome particles, the majority of the aerosol droplets having a diameter less than 5 $\mu$m and having an aerodynamic mass median diameter in the range of from 1 to 3 $\mu$m, the liposome particles being substantially homogeneous in size and having a diameter of less than 1 $\mu$m.

2.  Aqueous aerosol droplets as claimed in claim 1, which additionally contain one or more than one biologically active compound associated with the one or more liposome particles.

3.  Aqueous aerosol droplets as claimed in claim 2, wherein the or each biologically active compound is a drug selected from the group consisting of antiasthma, antiarrhythimic, antifungals, antihypertensives, anticancer, antibiotics, antidiabetics, antihistamines, antiparasitics, antivirals, cardiac glycosides, hormones, immunotherapies, antihypotensives, steriods, sedatives, and analgesics, tranquilizers, vaccines, and cell surface receptor blockers.

4.  Aqueous aerosol droplets as claimed in claim 2 or claim 3, wherein the or each biologically active compound is bound to liposomal bilayers of the liposomes.

5.  A method of producing the aerosol droplets of claims 1 to 4, comprising placing heterogeneous particles of the liposomes in an aqueous medium in an aerosol reservoir and aerosolizing the heterogeneous particles of liposomes with air effective to produce the aqueous aerosol droplets and to homogenize the heterogeneous particles of liposomes.

6.  A method as claimed in claim 5, wherein the aqueous aerosol droplets are produced using a nebulizer.

7.  A method as claimed in claim 5 or claim 6, wherein the heterogeneous particles of the liposomes are associated with one or more biologically active compounds.

8.  A method as claimed in claim 7, wherein one or more biologically active compounds are bound to liposomal bilayers of the heterogeneous liposome particles subjected to aerosolizing.

**Patentansprüche**

1.  Wässerige Aerosoltröpfchen, enthaltend eines oder mehrere Liposomteilchen, wobei die Mehrheit der Aerosoltröpfchen einen Durchmesser von weniger als 5 $\mu$m und einen aerodynamischen massemittleren Durchmesser im Bereich von 1 bis 3 $\mu$m aufweist, die Liposomteilchen im wesentlichen homogen in der Größe sind und einen Durchmesser von weniger als 1 $\mu$m haben.

2.  Wässerige Aerosoltröpfchen nach Anspruch 1, die zusätzlich eine oder mehr als eine biologisch wirksame Verbindung, verbunden mit einem oder mehreren Liposomteilchen, enthalten.

3.  Wässerige Aerosoltröpfchen nach Anspruch 2, wobei die oder jede biologisch wirksame Verbindung ein Arzneimittel ist, ausgewählt aus der Gruppe, bestehend aus Antiasthmamitteln, antiarrhythmischen,

11

antifungalen, antihypertensiven, Antikrebsmitteln, Antibiotika, Antidiabetesmitteln, Antihistaminika, antiparasitischen, antiviralen, Herzglycosiden, Hormonen, Immunotherapeutika, Antihypotensiva, Steroiden, Sedativa und Analgetika, Beruhigungsmitteln, Impfstoffen und Zelloberflächenrezeptorblockern.

4. Wässerige Aerosoltröpfchen nach Anspruch 2 oder 3, worin die oder jede biologisch wirksame Verbindung an liposomale Bilayer der Liposomen gebunden ist.

5. Verfahren zur Herstellung von Aerosoltröpfchen nach den Ansprüchen 1 bis 4, umfassend Anordnen der heterogenen Teilchen der Liposomen in einem wässerigen Medium in einem Aerosolreservoir und Zerstäuben der heterogenen Teilchen der Liposomen mit Luft unter Herstellung der wässerigen Aerosoltröpfchen und unter Homogenisierung der heterogenen Teilchen der Liposomen.

6. Verfahren nach Anspruch 5, wobei die wässerigen Aerosoltröpfchen unter Verwendung eines Zerstäubers hergestellt werden.

7. Verfahren gemäß Anspruch 5 oder Anspruch 6, wobei die heterogenen Teilchen der Liposomen mit einer oder mehreren biologisch wirksamen Verbindungen verbunden sind.

8. Verfahren nach Anspruch 7, wobei eine oder mehrere biologisch wirksame Verbindungen an liposomale Bilayer der zum Zerstäuben vorgesehenen heterogenen Liposomteilchen gebunden sind.

**Revendications**

1. Gouttelettes aqueuses d'aérosol contenant une ou plusieurs particules de liposome, la majorité des gouttelettes d'aérosol ayant un diamètre inférieur à 5 $\mu$m et ayant un diamètre médian massique aérodynamique dans la gamme de 1 à 3 $\mu$m, les particules de liposome étant de granulométrie essentiellement homogène et ayant un diamètre inférieur à 1 $\mu$m.

2. Gouttelettes aqueuses d'aérosol selon la revendication 1, qui contiennent en outre un ou plusieurs composés à activité biologique associés aux particules d'un ou plusieurs liposomes.

3. Gouttelettes aqueuses d'aérosol selon la revendication 2, dans lesquelles le ou chacun des composés à activité biologique est un médicament choisi dans le groupe constitué par les anti-asthmatiques, anti-arythmiques, les anti-fongiques, les anti-hypertenseurs, les anti-cancéreux, les antibiotiques, les anti-diabétiques, les anti-histaminiques, les anti-parasitaires, les anti-viraux, les glucosides cardiaques, les hormones, les immunothérapeutiques, les anti-hypotenseurs, les stéroïdes, les sédatifs et les analgésiques, les tranquillisants, les vaccins et les inhibiteurs superficiels des cellules.

4. Gouttelettes aqueuses d'aérosol selon la revendication 2 ou 3, dans lesquelles le ou chacun des composés à activité biologique est lié aux bi-couches liposomiques des liposomes.

5. Procédé de production des gouttelettes d'aérosol selon les revendications 1 à 4, comprenant le placement de particules hétérogènes des liposomes dans un milieu aqueux, dans un réservoir d'aérosol, et la formation d'un aérosol des particules hétérogènes de liposomes avec de l'air, efficace pour produire les gouttelettes aqueuses d'aérosol et pour homogénéiser les particules hétérogènes de liposomes.

6. Procédé selon la revendication 5, dans lequel les gouttelettes aqueuses d'aérosol sont produites à l'aide d'un nébulisateur.

7. Procédé selon la revendication 5 ou la revendication 6, dans lequel les particules hétérogènes des iiposomes sont associées à un ou plusieurs composés à activité biologique.

8. Procédé selon la revendication 7, dans lequel un ou plusieurs composés à activité biologique sont liés aux bicouches liposomiques des particules hétérogènes de liposome soumises à la formation d'un aérosol.